# EUROPÄISCHE PATENTANMELDUNG

(11) **EP 4 725 466 A2**
(43) Veröffentlichungstag der Anmeldung: **15.04.2026**
(21) Anmeldenummer: 25223209.5
(22) Anmeldetag: 12.12.2022
(51) Int. Cl.: A61F 13/511

(54) **WALZENANORDNUNG ZUR VEREDELUNG VON WICKELFÄHIGER, INSBESONDERE VORVERFESTIGTER BAHNWARE UND EINE ENTSPRECHENDE BAHNWARE**

(30) Priorität: 23.12.2021 DE 102021134589
(62) Teilanmeldung aus: 22844435.2
(71) Anmelder: Matthews International Corporation, Pittsburgh, Pennsylvania 15212 (US); Matthews International GmbH, 48691 Vreden (DE)
(72) Erfinder: THIEL, Klaus-Dieter Kurt, 41751 Viersen (DE); WANDKE, Achim, 47906 Kempen (DE); VON WIRTH, Achim, 41812 Erkelenz (DE)
(74) Vertreter: Holzwarth-Rochford, Andreas

(57) **Zusammenfassung**

Die Erfindung betrifft eine Walzenanordnung zur Veredelung einer wickelfiihigen, insbesondere vorverfestigten Bahnware, aufweisend eine erste und eine zweite Pragewalze, welche einen Walzenspalt zum Pragen der Bahnwarezwischen sich ausbilden; wobei die erste Walze eine Mehrzahl Vorspriinge zum Erzeugen von Strukturelementen in der Bahnware und die zweite Walze eine Mehrzahl Vertiefungen zumAufnehmen derVorspriinge aufweist; wobei ein zwischen der ersten und der zweiten Pragewalze gebildeter Walzenspaltabschnitt zum Erzeugen einer das Strukturelement jeweils zumindest abschnittsweise umgebenden Umrandung aus lokal verfestigter Bahnware ausgebildet ist, wobei der Walzenspaltabschnitt zumindest einen ersten konischen Verfestigungsabschnitt und zumindest einen im Wesentlichen parallel zu den Walzenachsen ausgebildeten Verfestigungsabschnitt aufweist.

## Beschreibung

Die vorliegende Erfindung betrifft eine Walzenanordnung zur Veredelung einer wickelfähigen, insbesondere vorverfestigten Bahnware, aufweisend eine erste und eine zweite Prägewalze, welche einen Walzenspalt zum Prägen der Bahnware zwischen sich ausbilden; wobei die erste Walze eine Mehrzahl Vorsprünge zum Erzeugen von Strukturelementen in der Bahnware und die zweite Walze eine Mehrzahl Vertiefungen zum Aufnehmen der Vorsprünge aufweist, wobei ein zwischen der Nadel und der Vertiefung gebildeter Walzenspaltabschnitt zum Erzeugen einer das Strukturelement jeweils zumindest abschnittsweise umgebenden Umrandung aus lokal verfestigter Bahnware ausgebildet ist, wobei der Walzenspaltabschnitt zumindest einen ersten konischen Verfestigungsabschnitt aufweist. Die Erfindung betrifft ferner eine entsprechende Bahnware.

Aus der Druckschrift US 2018/0 228 666 A1 ist bereits eine Vorrichtung zum Herstellen eines flüssigkeitsabsorbierenden Vliesstoffs mit dreidimensionalen Strukturen und Öffnungen bekannt. Die darin beschriebene Vorrichtung weist eine Mehrzahl konischer Nadeln auf, welche mit komplementären konischen Vertiefungen in Eingriff gebracht werden. Im dadurch gebildeten konischen Spalt erfährt das zu prägende Material zum einen eine Kompression, zum anderen erzeugt die Spitze der Nadel Öffnungen im Vliesstoff.

Die offenbarte Vorrichtung weist allerdings den Nachteil auf, dass der zu prägende Stoff bei der Erzeugung der Öffnungen nur unzureichend geklemmt wird, so dass das Material im Zuge der Erzeugung der Öffnungen im Spalt verrutschen kann und dadurch nur eine geringe Gleichmäßigkeit der Strukturen im geprägten Material erreicht werden kann. Ferner weisen die erzeugten Strukturen im Vliesstoff nur eine einheitliche Kompressionsintensität auf, so dass bei der Konfiguration des Vliesstoffs hinsichtlich Absorptionsfähigkeit und Stabilität stets nur eine Kompromisslösung in Betracht kommen kann.

Es ist daher die Aufgabe der Erfindung, eine Walzenanordnung beziehungsweise eine wickelfähige Bahnware derart zu verbessern, dass diese oder dieser verbesserte Produkteigenschaften aufweist und wirtschaftlicher herstellbar ist.

Die Aufgabe wird gelöst mit den Merkmalen der unabhängigen Ansprüche. Vorteilhafte Ausführungsformen der Erfindung sind Gegenstand der jeweils abhängigen Ansprüche.

Demgemäß ist vorgesehen, dass der Walzenspaltabschnitt zumindest einen im Wesentlichen parallel zu den Walzenachsen ausgebildeten Verfestigungsabschnitt aufweist. Die Erfindung weist den Vorteil auf, dass mittels des parallel zu den Walzenachsen ausgebildeten Verfestigungsabschnitts eine bessere Ausrichtung der Walzen zueinander im Produktionsprozess ermöglicht wird. Der parallel zu den Walzenachsen ausgebildete Abschnitt fixiert die Walzen zueinander und sorgt somit für einen selbstzentrierenden Effekt. Im Resultat können die Walzen mit höheren Geschwindigkeiten betrieben werden als die aus dem Stand der Technik bekannte Vorrichtung. Gleichzeitig sorgt der parallel zu den Walzenachsen ausgebildete Verfestigungsabschnitt für eine verbesserte Materialklemmung, so dass das Material nicht über die konischen Bereiche rutschen kann und daher die Öffnungen mit einer höheren Genauigkeit herstellbar sind. Ferner lassen sich durch den konischen Verfestigungsabschnitt einerseits und den parallel zu den Walzenachsen ausgebildeten Verfestigungsabschnitt andererseits Bereiche unterschiedlicher Kompression im Material erzeugen, so dass zum einen eine hohe Materialstabilität und zum anderen eine angenehme Haptik bzw. gute Absorptionseigenschaften realisiert werden können. Die Bahnware kann beispielsweise ein Vliesstoff sein. Der Vliesstoff kann hydrophil oder hydrophob sein. Die Bahnware kann ferner Tissue oder Folie sein. Die Bahnware kann einlagig, mehrlagig und/oder kaschiert sein.

Es kann vorgesehen sein, dass der Walzenspaltabschnitt ein Stufenprofil aufweist. Das Stufenprofil kann eine Mehrzahl konischer und eine Mehrzahl im Wesentlichen parallel zu den Walzenachsen ausgebildete Verfestigungsabschnitte aufweisen.

Es kann vorgesehen sein, dass der konische Verfestigungsabschnitt das Strukturelement ringförmig umschließt. Es ist ferner möglich, dass der im Wesentlichen parallel zu den Walzenachsen ausgerichtete Verfestigungsabschnitt den konischen Verfestigungsabschnitt zumindest abschnittsweise umgibt. Der im Wesentlichen parallel zu den Walzenachsen ausgerichtete Verfestigungsabschnitt kann sich auf der Ebene des Wälzkreisdurchmessers der ersten und der zweiten Prägewalze befinden. Der im Wesentlichen parallel zu den Walzenachsen ausgerichtete Verfestigungsabschnitt kann sich über die gesamte Walzenbreite erstrecken und überall dort vorgesehen sein, wo keine der Walzen einen Vorsprung oder eine Vertiefung aufweist.

Es ist denkbar, dass die Vorsprünge als Nadeln ausgebildet sind. Die Nadeln können zum Erzeugen von Öffnungen ausgebildet sein. Wenn die Vorsprünge als Nadeln ausgebildet sind, sind die Strukturelemente folglich als Öffnungen ausgebildet. Die Materialklemmung durch den konischen und den im Wesentlichen parallel zu den Walzenachsen ausgebildeten Verfestigungsabschnitt ermöglicht für die Nadeln eine zielgenaue und akkurate Erzeugung der Öffnungen in der Bahnware. Alternativ können die Vorsprünge als Prägeerhebungen ausgebildet sein, welche zum Erzeugen von als Erhebungen ausgebildeten Strukturelementen vorgesehen sind. Wenn die Vorsprünge als Prägeerhebungen ausgebildet sind, sind die Strukturelemente folglich als Erhebungen ausgebildet. Auch in diesem Fall können durch die gute Materialklemmung die Erhebungen sehr präzise in das Material eingebracht werden.

Es ist denkbar, dass der erste konische Verfestigungsabschnitt zwischen einem konischen proximalen Flankenabschnitt der Vorsprünge und einem konischen Mündungsabschnitt der Vertiefung ausgebildet ist. Der Vorsprung kann auf einem Sockelbereich angeordnet sein. Der Vorsprung kann sich ausgehend vom Sockelbereich radial von der Walzenoberfläche wegerstrecken. Der Sockelbereich kann eine größere Breite aufweisen als die größte Breite des Vorsprungs. Der proximale Flankenabschnitt der Vorsprünge kann in einem an den Sockelbereich angrenzenden Fußbereich der Vorsprünge vorgesehen sein. Die Vertiefung kann einen im Wesentlichen zylindrischen Abschnitt aufweisen, welcher sich in die Walzenoberfläche radial hineinerstreckt. In einem Mündungsbereich zur Walzenoberfläche kann die Vertiefung an ihrer Oberseite eine Fase aufweisen, welche den konischen Mündungsabschnitt der Vertiefung bildet. Der Vorsprung kann einen runden Querschnitt aufweisen. Der Vorsprung kann alternativ einen ovalen Querschnitt aufweisen. Der Vorsprung kann somit im Wesentlichen kegelförmig sein.

Es kann vorgesehen sein, dass der im Wesentlichen parallel zu den Walzenachsen ausgebildete Verfestigungsabschnitt zwischen einem im Wesentlichen parallel zu den Walzenachsen ausgebildeten Sattelbereich der Vorsprünge und einem im Wesentlichen parallel zu den Walzenachsen ausgebildeten Schulterbereich der Vertiefung ausgebildet ist. Der Sattelbereich kann durch eine Oberseite des Prägesockels gebildet sein. Der Sattelbereich kann sich vom Fuß der Vorsprünge bis zu einer Außenkante des Sockelbereichs erstrecken. Die Außenkante des Sockelbereichs kann eine Fase aufweisen. Der Schulterbereich kann sich horizontal, d. h. parallel zu den Walzenachsen, von der Oberkante des konischen Mündungsabschnitts wegerstrecken. Der Schulterbereich und der Sattelbereich können parallel zueinander ausgerichtet sein.

Es ist denkbar, dass der konische Verfestigungsabschnitt unmittelbar an den im Wesentlichen parallel zu den Walzenachsen ausgebildeten Verfestigungsabschnitt angrenzt. Damit kann der die Umrandung erzeugende Walzenspaltabschnitt in einem Übergangsbereich zwischen dem Fußbereich der Vorsprünge und des Sockelbereichs angeordnet sein.

Es ist denkbar, dass der Walzenspaltabschnitt ferner einen zweiten konischen Verfestigungsabschnitt aufweist, welcher gegenüberliegend zum ersten konischen Abschnitt an den parallel zu den Walzenachsen ausgebildeten Verfestigungsabschnitt angrenzt, wobei sich der erste und der zweite konische Verfestigungsabschnitt in entgegengesetzten Richtungen vom parallel zu den Walzenachsen ausgebildeten Verfestigungsabschnitt wegerstrecken. Der zweite konische Verfestigungsabschnitt kann durch die an der Außenkante des Prägesockels vorgesehenen Fase und einem an die Fase angrenzenden konischen Prägeerhebungsabschnitt gebildet sein.

Ferner kann vorgesehen sein, dass die zweite Walze eine Mehrzahl Prägeerhebungen zum Erzeugen von Erhebungen in der Bahnware und die erste Walze eine Mehrzahl Ausnehmungen zum Aufnehmen der Prägeerhebungen aufweist. Die zweite Walze kann eine Mehrzahl erster Prägeerhebungen mit einem ersten Querschnitt und eine Mehrzahl zweiter Prägeerhebungen mit einem zweiten Querschnitt aufweisen, wobei der zweite Querschnitt vom ersten Querschnitt abweicht. Der erste Querschnitt kann eine ovale Grundform aufweisen. Der zweite Querschnitt kann eine runde Grundform aufweisen. Die Prägeerhebungen können angewinkelte Seitenwände und eine flache Oberseite aufweisen. Zwischen den Seitenwänden und der flachen Oberseite kann ein Radius von 0,3 mm vorgesehen sein.

Außerdem kann vorgesehen sein, der zweite konische Verfestigungsabschnitt zwischen einem konischen proximalen Flankenabschnitt der Prägeerhebung und einem konischen Mündungsabschnitt der Ausnehmung ausgebildet ist.

Es ist denkbar, dass eine Walzenspaltdicke im im Wesentlichen parallel zu den Walzenachsen ausgebildeten Verfestigungsabschnitt zwischen 0,01 mm und 0,03 mm, bevorzugt 0,02 mm beträgt. Es kann vorgesehen sein, dass die Walzenspaltdicke im im Wesentlichen parallel zu den Walzenachsen ausgebildeten Verfestigungsabschnitt kleiner ist als die Walzenspaltdicke im ersten und/oder zweiten konischen Verfestigungsabschnitt. Eine kleinere Walzenspaltdicke im im Wesentlichen parallel zu den Walzenachsen ausgebildeten Walzenspaltabschnitt sorgt für eine gute Klemmung des Materials und für eine höhere Kompression. Dadurch wird im parallel zu den Walzenachsen ausgebildeten Verfestigungsabschnitt eine höhere Materialstabilität erzeugt.

Beispielsweise kann vorgesehen sein, dass eine Walzenspaltdicke im ersten und/oder im zweiten konischen Verfestigungsabschnitt zwischen 0,02 mm und 0,08 mm, bevorzugt 0,05 mm beträgt.

Ferner kann eine Höhe des ersten konischen Verfestigungsabschnitts zwischen 0,2 mm und 0,6 mm, bevorzugt 0,4 mm betragen. Dadurch weist die Öffnung im Material eine kreisförmige konische Ringstruktur aus verfestigtem Material auf, welches die Öffnung umgibt.

Außerdem kann eine Höhe des zweiten konischen Verfestigungsabschnitts zwischen 0,2 mm und 0,6 mm, bevorzugt 0,4 mm betragen. Dadurch weist die Prägestruktur im Material eine kreisförmige konische Ringstruktur aus verfestigtem Material auf, welches die Prägestruktur umgibt.

Es kann vorgesehen sein, dass eine Breite des im Wesentlichen parallel zu den Walzenachsen ausgebildeten Verfestigungsabschnitts zwischen 0,5 und 1 mm, bevorzugt 0,7 mm beträgt. Die parallel zur Materialebene verfestigten Materialbereiche, welche die Öffnungen krempenförmig umgeben, sorgen ferner dafür, dass die dreidimensionale Materialstruktur der Bahnware widerstandsfähiger ist. Durch Einstellen der Größe des im Wesentlichen parallel zur Materialebene ausgebildeten Verfestigungsabschnitts kann somit eine Grundfestigkeit der herzustellenden Bahnware konfiguriert werden.

Es ist denkbar, dass ein Winkel des ersten konischen Verfestigungsabschnitts zur Vertikalen zwischen 10° und 18°, bevorzugt 14° beträgt.

Außerdem kann vorgesehen sein, dass ein Winkel des zweiten konischen Verfestigungsabschnitts zur Vertikalen zwischen 10° und 18°, bevorzugt 14° beträgt.

Ferner kann vorgesehen sein, dass der erste konische Verfestigungsabschnitt parallel zum zweiten konischen Verfestigungsabschnitt ausgerichtet ist.

Darüber hinaus kann vorgesehen sein, dass in der ersten und/oder in der zweiten Prägewalze eine Einrichtung zum Beheizen der Walzenoberfläche vorgesehen ist. Durch Einstellen einer optimalen Prägetemperatur in Verbindung mit einem vorbestimmten Prägedruck kann besonders vorteilhaft die gewünschte dreidimensionale Bahnwarenstruktur bei einer hohen Prozessgeschwindigkeit hergestellt werden.

Die Erfindung betrifft ferner eine wickelfähige, insbesondere vorverfestigte Bahnware, welche aufweist:
zumindest eine sich in einer Ebene erstreckende vorverfestigte Bahnwarenlage;
eine Mehrzahl auf der Bahnwarenlage verteilte Strukturelemente;
wobei die Strukturelemente jeweils eine das Strukturelement zumindest abschnittsweise umgebende Umrandung aufweisen, welche durch eine lokale Verfestigung der Bahnware ausgebildet ist, wobei die Umrandung zumindest einen ersten konischen Abschnitt und zumindest einen im Wesentlichen parallel zu der Ebene ausgebildeten Abschnitt aufweist.

Die Umrandung kann ein Stufenprofil aufweisen. Das Stufenprofil kann eine Mehrzahl konischer Abschnitte und eine Mehrzahl im Wesentlichen parallel zu der Ebene ausgebildete Abschnitte aufweisen.

Es kann vorgesehen sein, dass der erste konische Abschnitt unmittelbar an die Öffnung angrenzt. Ferner kann der im Wesentlichen parallel zu der Ebene ausgebildete Abschnitt unmittelbar an den ersten konischen Abschnitt angrenzen.

Außerdem kann vorgesehen sein, dass die Umrandung einen zweiten konischen Abschnitt aufweist, welcher gegenüberliegend zum ersten konischen Abschnitt an den im Wesentlichen parallel zu der Ebene ausgebildeten Abschnitt angrenzt. Dabei kann vorgesehen sein, dass der erste konische Abschnitt das Strukturelement ringförmig umschließt. Alternativ kann der im Wesentlichen parallel zu der Ebene ausgebildete Abschnitt den ersten konischen Abschnitt abschnittsweise umgeben. Die Strukturelemente können als Öffnungen ausgebildet sein. Die Strukturelemente können als Erhebungen ausgebildet sein. Der im Wesentlichen parallel zu der Ebene ausgebildete Abschnitt kann überall dort auf der Bahnwarenlage vorgesehen sein, wo diese kein Strukturelement wie Erhebungen, Vertiefungen oder Öffnungen aufweist.

Darüber hinaus kann vorgesehen sein, dass die Bahnware ferner eine Mehrzahl sich von der Ebene wegerstreckende Erhebungen aufweist.

Außerdem kann vorgesehen sein, dass der zweite konische Abschnitt ferner unmittelbar an eine Erhebung angrenzt.

Beispielhafte Ausführungsformen der Erfindung werden anhand der nachfolgenden Figuren erläutert. Dabei zeigt:
- Fig. 1: eine Frontalansicht einer Ausführungsform der erfindungsgemäßen Walzenanordnung;
- Fig. 2: eine Draufsicht auf einen Walzenabschnitt einer Ausführungsform der ersten Prägewalze;
- Fig. 3: eine vergrößerte Querschnittsansicht einer Ausführungsform der ersten Prägewalze;
- Fig. 4: eine Draufsicht auf einen Walzenabschnitt einer Ausführungsform der zweiten Prägewalze;
- Fig. 5: eine vergrößerte Querschnittsansicht einer Ausführungsform der zweiten Prägewalze; und

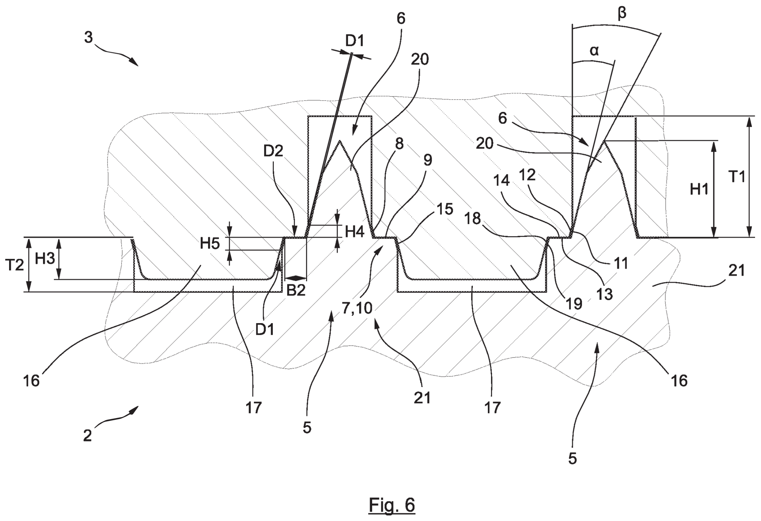

- Fig. 6: eine vergrößerte Querschnittsansicht einer Ausführungsform des zwischen der ersten und der zweiten Prägewalze ausgebildeten Prägespalts;
- Fig. 7: eine beispielhafte Ausführungsform einer wickelfähigen, insbesondere vorverfestigten, Bahnware in einer Querschnittsansicht;
- Fig. 8: eine vergrößerte Querschnittsansicht einer weiteren Ausführungsform des zwischen der ersten und der zweiten Prägewalze ausgebildeten Prägespalts;
- Fig. 9: eine vergrößerte Querschnittsansicht einer weiteren Ausführungsform des zwischen der ersten und der zweiten Prägewalze ausgebildeten Prägespalts.

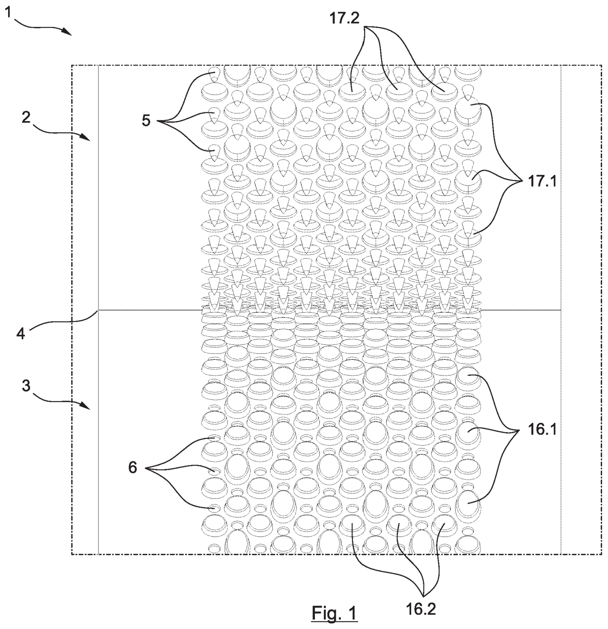

Die in Fig. 1 dargestellte Walzenanordnung 1 zeigt die einen Walzenspalt 4 ausbildenden erste Prägewalze 2 und zweite Prägewalze 3. Es ist zu erkennen, dass auf der ersten Prägewalze 2 eine Mehrzahl Nadeln 5 in regelmäßiger Verteilung angeordnet ist. Die Nadeln 5 weisen dabei zu allen benachbarten Nadeln 5 jeweils denselben Abstand auf. Selbstverständlich können die Nadeln 5 auch unterschiedliche Abstände zueinander aufweisen. Zwischen den Nadeln sind eine Mehrzahl erster Ausnehmungen 17.1 mit ovalem Querschnitt und eine Mehrzahl zweiter Ausnehmungen 17.2 mit rundem Querschnitt angeordnet. Die Anzahl erster zu zweiter Ausnehmungen 17.1, 17.2 beträgt 1:3. Auf der zweiten Prägewalze 3 sind zu den Strukturen der ersten Prägewalze komplementäre Prägestrukturen ausgebildet. Zum einen weist die zweite Prägewalze eine Mehrzahl Vertiefungen 6 auf, welche so ausgerichtet sind, dass beim Abrollen der Walzen 2, 3 aufeinander die Nadeln 5 in den Vertiefungen 6 aufgenommen werden. In ebenso komplementärer Anordnung weist die zweite Prägewalze ferner eine Mehrzahl erster Prägeerhebungen 16.1 und eine Mehrzahl Prägeerhebungen 16.2 auf, welche im Walzenspalt 4 in die entsprechenden Ausnehmungen 17.1, 17.2 eingreifen. Entsprechend zu den Ausnehmungen 17.1, 17.2 weisen die ersten Prägeerhebungen 16.1 einen ovalen Querschnitt und die zweiten Prägeerhebungen 16.2 einen runden Querschnitt auf.

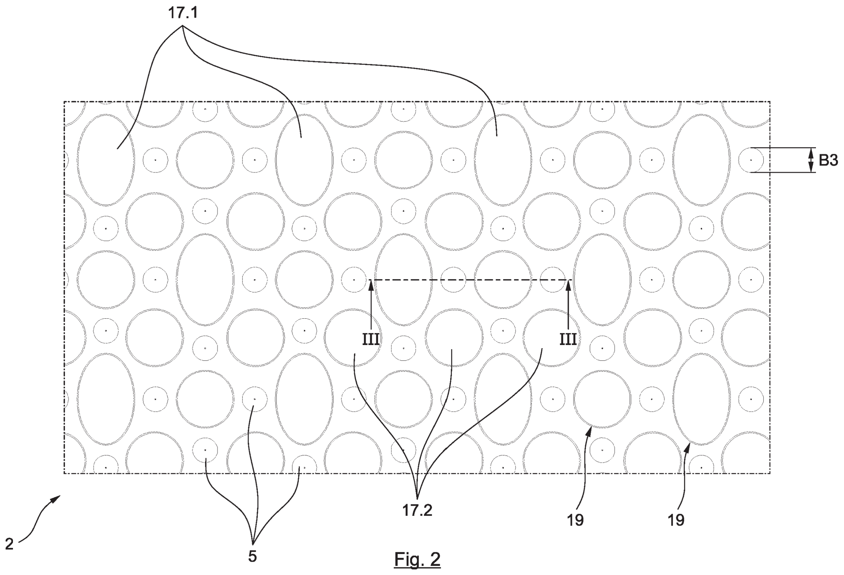

Fig. 2 zeigt eine Draufsicht auf die Prägestruktur der ersten Prägewalze 2. Die Prägestruktur weist eine Mehrzahl Nadeln 5 auf, ferner eine Mehrzahl erster Ausnehmungen 17.1 mit ovalem Querschnitt und eine Mehrzahl zweiter Ausnehmungen 17.2 mit rundem Querschnitt auf. Es ist zu erkennen, dass jede Nadel 5 jeweils von drei zweiten Ausnehmungen 17.2 mit rundem Querschnitt und einer ersten Ausnehmung 17.1 mit ovalem Querschnitt umgeben ist. Dabei kann die erste Ausnehmung 17.1 mit ovalem Querschnitt links, rechts, oberhalb oder unterhalb der Nadeln 5 angeordnet sein. Die größte Breite B3 der Nadeln 5, das heißt, die Breite des an den Sockelbereich 21 angrenzenden Fußbereichs der Nadeln 5, beträgt in der dargestellten Ausführungsform 2,2 mm. Die ersten und die zweiten Ausnehmungen 17.1, 17.2 weisen jeweils an ihrer zur Walzenoberfläche weisenden Oberkante einen konischen Mündungsabschnitt 19 auf, welcher in Form einer die Ausnehmungen ringförmig umgebenden Fase ausgebildet ist. Die Breite des konischen Mündungsabschnitts 19 beider Ausnehmungen 17.1, 17.2 beträgt in der dargestellten Ausführungsform 0,2 mm.

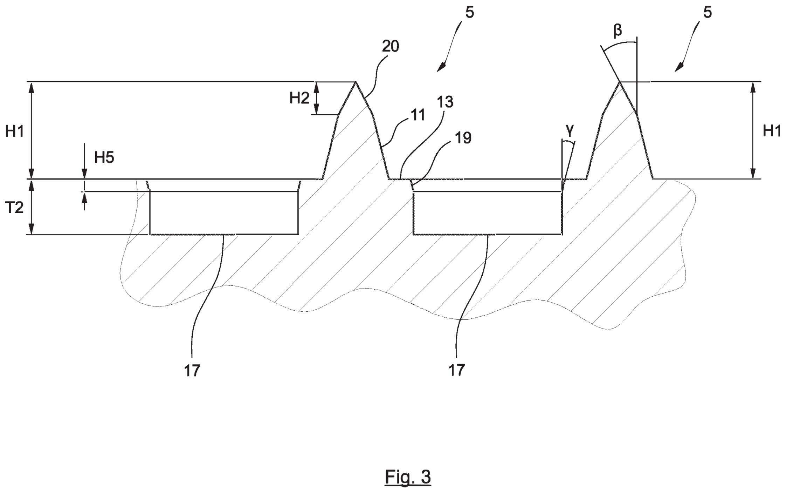

Fig. 3 zeigt eine vergrößerte Querschnittsansicht der Prägewalzenstruktur der ersten Prägewalze 2. Dabei ist insbesondere eine Querschnittsansicht der Nadeln 5 zu erkennen. Die Nadel 5 ist auf einem Sockelbereich 21 angeordnet und weist im Wesentlichen eine sich vom Sockelbereich 21 konisch bzw. kegelförmig wegerstreckende Form auf. Der Sockelbereich weist eine größere Breite auf als die Breite B3 des Fußbereichs der Nadeln 5. Die Oberseite des Sockelbereichs 21 liegt dabei auf der Ebene des Wälzkreisdurchmessers der Walzen. Die Höhe H1 der Nadeln 5 beträgt in der dargestellten Ausführungsform 3,2 mm. An der Oberseite der Nadeln 5 befindet sich eine Nadelspitze 20 mit einer Höhe H2, welche in der dargestellten Ausführungsform 1,07 mm beträgt. Die Nadelspitze 20 läuft mit einem Flankenwinkel β weniger spitz zu als der untere Bereich der Nadeln 5, welcher einen Flankenwinkel a aufweist, wobei β > α. Im dargestellten Beispiel beträgt α 14° und β 28°. Der untere Bereich der Nadeln 5 stellt einen konisch proximalen Flankenabschnitt 11 bereit, in welchem der erste konische Abschnitt der Umrandung der Öffnung der Bahnwareerzeugt wird. Der im Wesentlichen horizontale Sattelbereich 13 der Nadeln 5 wird durch die horizontal über den Nadelquerschnitt auskragenden Oberseiten des Sockelbereichs 21 gebildet und dient zur Herstellung des im Wesentlichen horizontal ausgebildeten Verfestigungsabschnitt 9 der Umrandung. An den Sattelbereich 13 grenzen konische Mündungsabschnitte 19 der vier den Nadel 5 umgebenden Ausnehmungen 17.1, 17.2 an. Diese bilden mit den jeweils komplementären Prägeerhebungen 16.1, 16.2 dann jeweils abschnittsweise den zweiten konischen Verfestigungsabschnitt 15. Die Ausnehmungen 17 weisen jeweils eine Tiefe T2 auf, welche im dargestellten Beispiel 1,8 mm beträgt. Die Höhe H5 des konischen Mündungsabschnitts 19 der Ausnehmungen 17 beträgt in der dargestellten Ausführungsform jeweils 0,4 mm. Der konische Mündungsabschnitt weist einen Winkel γ gegenüber der Vertikalen auf, welcher im dargestellten Beispiel 14° beträgt.

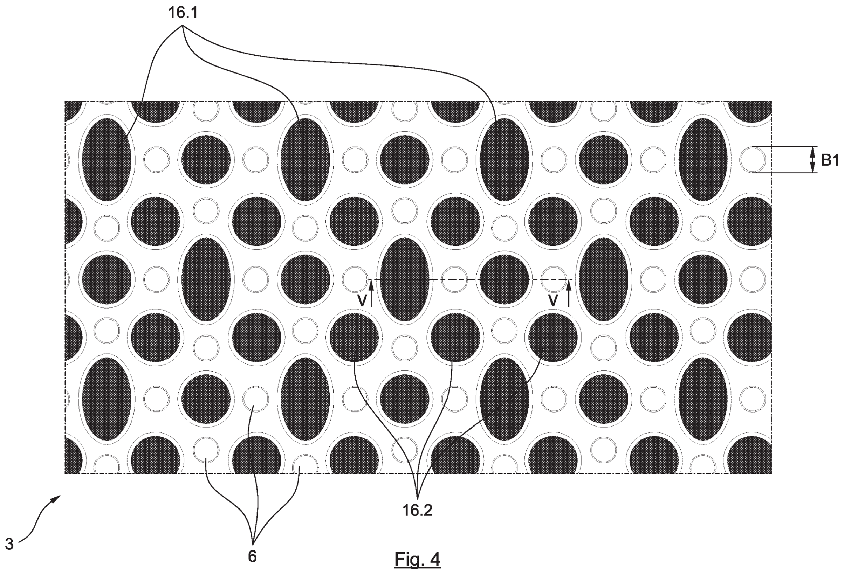

Fig. 4 zeigt eine Draufsicht auf die Prägestruktur der zweiten Prägewalze 3. Die Prägestruktur weist eine Mehrzahl Vertiefungen 6 auf, ferner eine Mehrzahl erster Prägeerhebungen 16.1 mit ovalem Querschnitt und eine Mehrzahl zweiter Prägeerhebungen 16.2 mit rundem Querschnitt auf. Entsprechend zu Fig. 2 ist zu erkennen, dass jede Vertiefung 6 jeweils von drei zweiten Prägeerhebungen 16.2 mit rundem Querschnitt und einer ersten Prägeerhebung 16.1 mit ovalem Querschnitt umgeben ist. Dabei kann die erste Prägeerhebung 16.1 mit ovalem Querschnitt links, rechts, oberhalb oder unterhalb der Nadeln 5 angeordnet sein. Die Breite B1 der Vertiefung 6, das heißt, die Breite des sich in die Walzenoberfläche hineinerstreckenden Vertiefungszylinders, beträgt in der dargestellten Ausführungsform 2,1 mm. Die ersten und die zweiten Prägeerhebungen 16.1, 16.2 weisen jeweils an ihrer von der Walzenoberfläche wegweisenden Oberkante eine Abrundung auf, welche in der dargestellten Ausführungsform einen Radius von 0,3 mm aufweist. Die Breite des konischen Mündungsabschnitts 12 der Vertiefung 6 beträgt in der dargestellten Ausführungsform 0,2 mm.

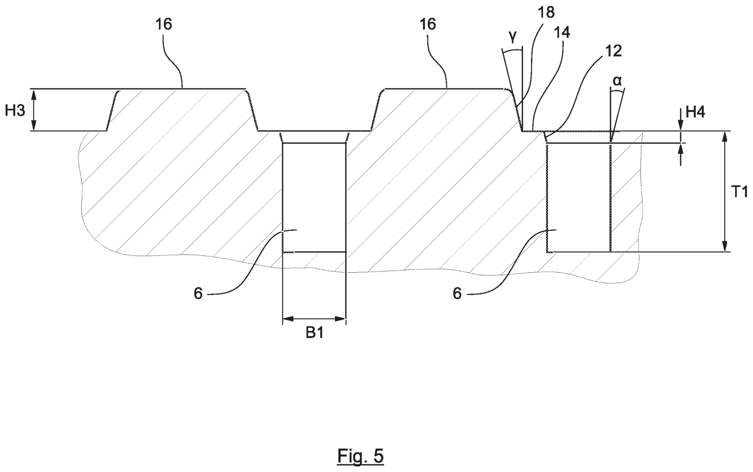

Fig. 5 zeigt eine vergrößerte Querschnittsansicht der Prägewalzenstruktur der zweiten Prägewalze 3. Dabei ist insbesondere eine Querschnittsansicht der Vertiefungen 6 zu erkennen. Die Vertiefungen 6 sind zwischen den Prägeerhebungen 16 angeordnet und weisen im Wesentlichen eine sich in die Walzenoberfläche hineinerstreckende zylindrische Form mit einer Breite B1 und einer Tiefe T1 auf, wobei in der dargestellten Ausführungsform die Breite B1 2,1 mm und die Tiefe T1 4 mm beträgt. An der Oberkante des Mündungsbereichs der Vertiefung 6 weist diese eine Breite von 2,3 mm auf. Damit ist diese Breite größer als die größte Breite B3 der Nadeln 5 und die Tiefe T1 ist größer als die Höhe H1 der Nadeln 5. Der Miindungswinkel des Mündungsbereichs 12 weist einen Winkel α von 14° auf. Der die Oberkante der Vertiefung umgebende Schulterbereich 14 liegt auf der Ebene des Wälzkreisdurchmessers der Walzen. Der konische Mündungsbereich 12 der Vertiefung 6 dient im Zusammenwirken mit dem konisch proximalen Flankenabschnitt 11 der Nadeln zum Erzeugen des ersten konischen Abschnitts der Umrandung der Öffnung der Bahnware. Gemeinsam mit dem horizontalen Sattelbereich 13 der Nadeln 5 dient der im Wesentlichen horizontale Schulterbereich 14 der Vertiefung 6 zur Herstellung des im Wesentlichen horizontal ausgebildeten Verfestigungsabschnitt 9 der Umrandung. An den Schulterbereich 14 grenzen konische Flankenabschnitte 18 der Prägeerhebungen 16 der vier die Vertiefung 6 umgebenden Prägeerhebungen 16.1, 16.2 an. Deren proximale Bereiche bilden mit den jeweils komplementären Ausnehmungen 17.1, 17.2 dann jeweils abschnittsweise den zweiten konischen Verfestigungsabschnitt 15. Die Prägeerhebungen 16 weisen jeweils eine Höhe H3 auf, welche im dargestellten Beispiel 1,4 mm beträgt. Die konischen Flankenabschnitte 18 der Prägeerhebungen weisen jeweils einen Winkel γ gegenüber der Vertikalen auf, welcher im dargestellten Beispiel 14° beträgt.

Fig. 5 zeigt schließlich eine vergrößerte Querschnittsansicht einer Ausführungsform des zwischen der ersten und der zweiten Prägewalze 2 ,3 ausgebildeten Prägespalts 4. Beim Abrollen der Prägewalzen 2, 3 aufeinander greifen beim Durchlaufen des Prägespalts 4 die Prägeerhebungen 16 der zweiten Prägewalze 3 in die Ausnehmungen 17 der ersten Prägewalze 2. In den Bereichen des Aufeinandertreffens der konisch proximalen Flankenabschnitte 18 der Prägeerhebungen 16 und der konischen Mündungsabschnitte 19 der Ausnehmungen 17, welche parallel zueinander ausgerichtet sind, wird der zweite konische Verfestigungsabschnitt 15 gebildet. Dieser weist eine Höhe H5 auf. Der Verfestigungsabschnitt 15 wird an der Oberseite durch den im Wesentlichen horizontalen Schulterbereich 14 der Vertiefung 6 begrenzt und an der Unterseite dadurch, dass sich der Prägespalt durch den unterhalb zum Verfestigungsabschnitt 15 anschließenden vertikalen Verlauf der Seitenwand der Ausnehmung 17 aufweitet. Der Verfestigungsabschnitt 15 weist eine Prägespaltdicke D1 von 0,05 mm auf.

Beim Abrollen der Prägewalzen 2, 3 aufeinander greifen ferner beim Durchlaufen des Prägespalts 4 die Nadeln 5 der ersten Prägewalze 2 in die Vertiefungen 6 der zweiten Prägewalze 3. In den Bereichen des Aufeinandertreffens der im Wesentlichen horizontalen Sattelbereiche 13 des Sockelbereichs 21 und den im Wesentlichen horizontalen Schulterbereichen 14 der Vertiefungen 6, welche parallel zueinander ausgerichtet sind, wird der im Wesentlichen horizontale Verfestigungsabschnitt 9 ausgebildet. Dieser weist eine Breite B2 auf. Der Verfestigungsabschnitt 9 wird an der dem Nadel 5 abgewandten Seite durch den konisch proximalen Flankenabschnitt 18 der Prägeerhebung 16 begrenzt und an der der dem Nadel 5 zugewandten Seite durch den konisch proximalen Flankenabschnitt 11 der Nadeln 5. Der Verfestigungsabschnitt 9 weist eine Prägespaltdicke D2 von 0,02 mm auf und erzeugt somit eine größere Klemmung und eine größere Materialkompression als die konischen Verfestigungsabschnitte 15 und 8.

In den Bereichen des Aufeinandertreffens der konisch proximalen Flankenabschnitte 11 der Nadeln 5 und den konischen Mündungsabschnitten 12 der Vertiefung 6, welche parallel zueinander ausgerichtet sind, wird der erste konische Verfestigungsabschnitt 8 ausgebildet. Dieser weist eine Höhe H4 auf. Der Verfestigungsabschnitt 8 wird an seine Unterseite durch den im Wesentlichen horizontalen Sattelbereich 13 des Sockelbereichs 21 begrenzt und an seiner Oberseite dadurch, dass sich der Prägespalt durch den oberhalb zum Verfestigungsabschnitt 8 anschließenden vertikalen Verlauf der Seitenwand der Vertiefung 6 aufweitet. Der erste konische Verfestigungsabschnitt 8 weist wie der zweite konische Verfestigungsabschnitt 15 eine Prägespaltdicke D2 von 0,05 mm auf. Die aneinander angrenzenden Verfestigungsabschnitte 8, 9, 15 bilden ein Stufenprofil 10.

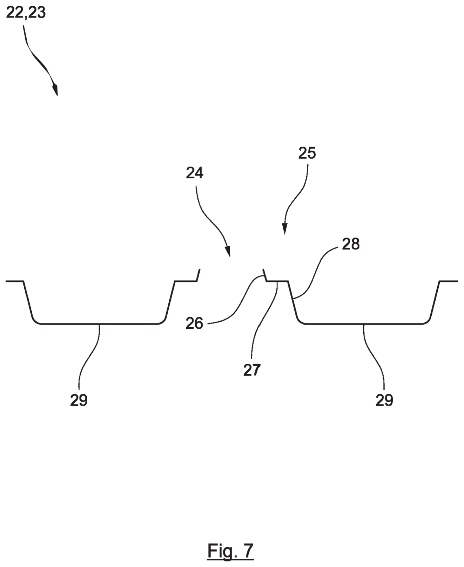

Fig. 7 zeigt eine beispielhafte Ausführungsform einer wickelfähigen, insbesondere vorverfestigten, Bahnware 22 in einer Querschnittsansicht. Die Bahnware 22 weist eine sich in einer Ebene erstreckende wickelfähige Bahnwarenlage 23 auf, welche eine Mehrzahl sich durch die Bahnwarenlage 23 erstreckende Öffnungen 24 aufweist. Die Öffnungen 24 weisen jeweils eine die Öffnung 24 umgebende Umrandung 25 auf, welche sich durch eine lokale Verfestigung der Bahnware 22 auszeichnet. Die Umrandung 25 weist dabei einen ersten konischen Abschnitt 26 und einen im Wesentlichen parallel zu der Ebene ausgebildeten Abschnitt 27 auf. Ferner weist die Umrandung 25 einen zweiten konischen Abschnitt 28 auf, welcher gegenüberliegend zum ersten konischen Abschnitt 26 an den im Wesentlichen parallel zu der Ebene ausgebildeten Abschnitt 27 angrenzt. Dadurch weist die Umrandung 25 ein Stufenprofil auf. Der erste konische Abschnitt 26 grenzt unmittelbar an die Öffnung 24 an. Der im Wesentlichen parallel zu der Ebene ausgebildete Abschnitt 27 grenzt ferner unmittelbar an den ersten konischen Abschnitt 26 an. Die Bahnware weist ferner eine Mehrzahl sich von der Ebene wegerstreckende Erhebungen 29 auf, wobei der zweite konische Abschnitt 28 unmittelbar an eine benachbarte Erhebung 29 angrenzt.

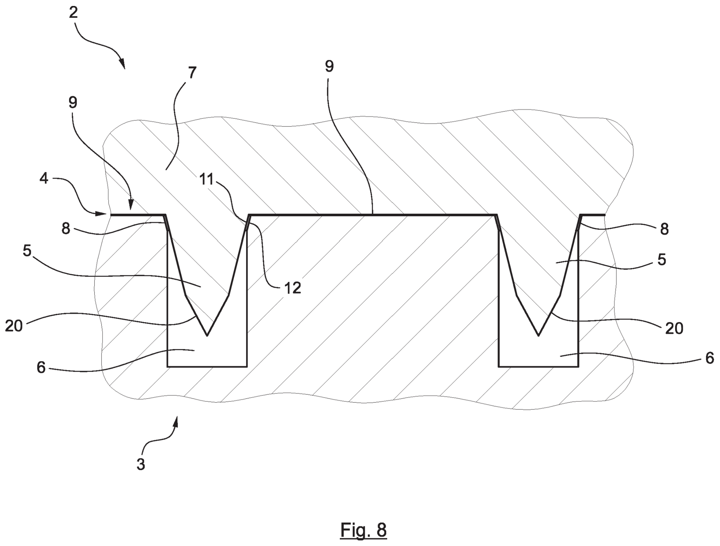

In Fig. 8 ist eine weitere Ausführungsform der Erfindung dargestellt. Bei dieser weist die erste Prägewalze 2 als Strukturelemente ausschließlich Nadeln 5 und die zweite Prägewalze 3 ausschließlich entsprechende Vertiefungen 6 auf. Beim Eingreifen der Nadeln 5 in die Vertiefungen 6 wird im Walzenspalt 4 die Bahnware verfestigt bzw. perforiert. Dabei findet in den horizontalen Verfestigungsabschnitten 9 sowie in den an diese angrenzenden und die Nadeln 5 ringförmig umschließenden ersten konischen Verfestigungsabschnitten 8 im Fußbereich der Nadeln 5 eine Klemmung des zu perforierenden Materials statt, so dass die Nadelspitzen 20 präzise die Öffnungen im Material erzeugen können. Auch bei dieser Ausführungsform wird der erste konische Verfestigungsabschnitt 8 zwischen dem konisch proximalen Flankenabschnitt 11 der Nadeln 5 und dem konischen Mündungsabschnitt der Vertiefung 6 gebildet. Der im Wesentlichen parallel zu den Walzenachsen ausgebildete Verfestigungsabschnitt 9 wird im durch die Wälzkreisdurchmesser der ersten und der zweiten Prägewalze 2, 3 gebildeten Walzenspaltabschnitt erzeugt, welcher sich überall dort über die komplette Walzenbreite erstreckt, wo keine Nadeln 5 oder Vertiefungen 6 angeordnet sind.

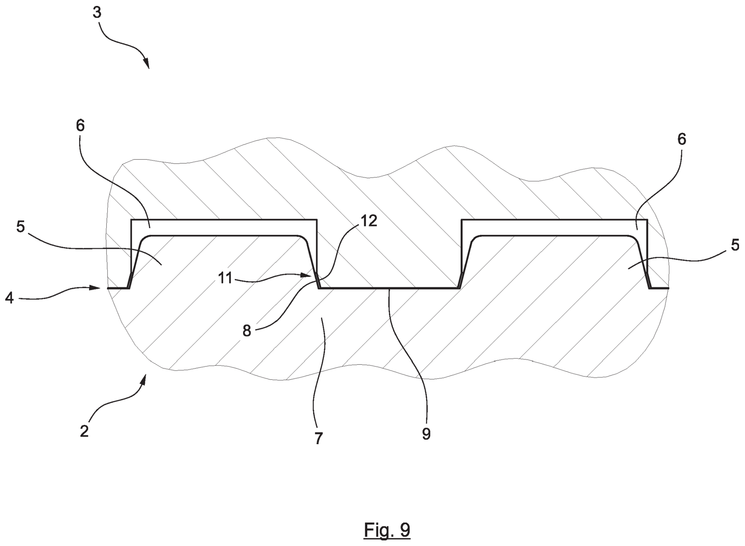

Fig. 9 zeigt noch eine weitere Ausführungsform der Erfindung, bei welcher die erste Prägewalze 2 als Strukturelemente ausschließlich Prägeerhebungen 5 aufweist und die zweite Prägewalze 3 als Strukturelemente ausschließlich entsprechende Vertiefungen 6 aufweist. Die Prägeerhebungen 5 greifen beim Durchlaufen des Walzenspalts 4 zwar in die Vertiefungen 6 ein, allerdings erzeugen diese anstelle von Öffnungen lediglich Erhebungen bzw. Ausbuchtungen im zu strukturierenden Material. Die Prägeerhebungen 5 weisen jeweils konische Flanken auf, deren konisch proximale Abschnitte 11 im Fußbereich der Prägeerhebungen 5 gemeinsam mit den konischen Mündungsabschnitten 12 der Vertiefung 6 jeweils den ersten konischen Verfestigungsabschnitt 8 des Walzenspalts 4 bilden. Der konische Verfestigungsbereich 8 umschließt dabei die gesamte Prägeerhebung 5 ringförmig. Der im Wesentlichen parallel zu den Walzenachsen ausgebildete Verfestigungsabschnitt 9 wird entsprechend zur Ausführungsform in Fig. 3 im durch die Wälzkreisdurchmesser der ersten und der zweiten Prägewalze 2, 3 gebildeten Walzenspaltabschnitt erzeugt, welcher sich überall dort über die komplette Walzenbreite erstreckt, wo keine Prägeerhebungen 5 oder Vertiefungen 6 angeordnet sind.

Die in der vorstehenden Beschreibung, in den Figuren sowie in den Ansprüchen offenbarten Merkmale der Erfindung können sowohl einzeln als auch in beliebiger Kombination für die Verwirklichung der Erfindung wesentlich sein.

Weitere nicht einschränkende Ausführungsformen oder Aspekte sind in den folgenden Beispielen dargelegt.

### Beispiele

1. Walzenanordnung (1) zur Veredelung einer wickelfähigen, insbesondere vorverfestigten Bahnware, aufweisend
   eine erste und eine zweite Prägewalze (2, 3), welche einen Walzenspalt (4) zum Prägen der Bahnware zwischen sich ausbilden;
   wobei die erste Walze (2) eine Mehrzahl Vorsprünge (5) zum Erzeugen von Strukturelementen in der Bahnware und die zweite Walze (3) eine Mehrzahl Vertiefungen (6) zum Aufnehmen der Vorsprünge (5) aufweist;
   wobei ein zwischen der ersten und der zweiten Prägewalze (2, 3) gebildeter Walzenspaltabschnitt (7) zum Erzeugen einer das Strukturelement (24) jeweils zumindest abschnittsweise umgebenden Umrandung aus lokal verfestigter Bahnware ausgebildet ist, wobei der Walzenspaltabschnitt (7) zumindest einen ersten konischen Verfestigungsabschnitt (8) aufweist, dadurch gekennzeichnet, dass der Walzenspaltabschnitt (7) ferner zumindest einen im Wesentlichen parallel zu den Walzenachsen ausgebildeten Verfestigungsabschnitt (9) aufweist.
2. Walzenanordnung (1) nach Beispiel 1, wobei der Walzenspaltabschnitt (7) ein Stufenprofil (10) aufweist.
3. Walzenanordnung (1) nach Beispiel 1 oder 2, wobei der erste konische Verfestigungsabschnitt (8) zwischen einem konischen proximalen Flankenabschnitt (11) der Vorsprünge (5) und einem konischen Mündungsabschnitt (12) der Vertiefung (6) ausgebildet ist.
4. Walzenanordnung (1) nach einem derBeispiele 1 bis 3, wobei der im Wesentlichen parallel zu den Walzenachsen ausgebildete Verfestigungsabschnitt (9) zwischen einem im Wesentlichen parallel zu den Walzenachsen ausgerichteten Sattelbereich (13) der Vorsprünge (5) und einem im Wesentlichen parallel zu den Walzenachsen ausgerichteten Schulterbereich (14) der Vertiefung (6) ausgebildet ist.
5. Walzenanordnung (1) nach einem der vorangehenden Beispiele, wobei der erste konische Verfestigungsabschnitt (8) unmittelbar an den im Wesentlichen parallel zu den Walzenachsen ausgebildeten Verfestigungsabschnitt (9) angrenzt.
6. Walzenanordnung (1) nach Beispiel 5, wobei der erste konische Verfestigungsabschnitt (8) das Strukturelement (5) ringförmig umschließt.
7. Walzenanordnung (1) nach Beispiel 5, wobei der im Wesentlichen parallel zu den Walzenachsen ausgerichtete Verfestigungsabschnitt (9) den ersten konischen Verfestigungsabschnitt (8) abschnittsweise umgibt.
8. Walzenanordnung (1) nach einem der vorangehenden Beispiele, wobei die Vorsprünge (5) Nadeln sind, welche zum Erzeugen von als Öffnungen ausgebildeten Strukturelementen vorgesehen sind.
9. Walzenanordnung (1) nach einem der Beispiele 1 bis 7, wobei die Vorsprünge (5) Prägeerhebungen (16) sind, welche zum Erzeugen von als Erhebungen ausgebildeten Strukturelementen vorgesehen sind.
10. Walzenanordnung (1) nach einem der vorangehenden Beispiele, wobei der Walzenspaltabschnitt (7) ferner einen zweiten konischen Verfestigungsabschnitt (15) aufweist, welcher gegenüberliegend zum ersten konischen Verfestigungsabschnitt (8) an den 'im Wesentlichen parallel zu den Walzenachsen ausgebildeten Verfestigungsabschnitt (9) angrenzt, wobei sich der erste und der zweite konische Verfestigungsabschnitt (8, 15) in entgegengesetzten Richtungen vom im Wesentlichen parallel zu den Walzenachsen ausgebildeten Verfestigungsabschnitt (9) wegerstrecken.
11. Walzenanordnung (1) nach einem der vorangehenden Beispiele, wobei die zweite Walze (3) eine Mehrzahl Prägeerhebungen (16) zum Erzeugen von Erhebungen in der Bahnware und die erste Walze (2) eine Mehrzahl Ausnehmungen (17) zum Aufnehmen der Prägeerhebungen (16) aufweist.
12. Walzenanordnung (1) nach einem der Beispiele 10 oder 11, wobei der zweite konische Verfestigungsabschnitt (15) zwischen einem konischen proximalen Flankenabschnitt (18) der Prägeerhebung (16) und einem konischen Mündungsabschnitt (19) der Ausnehmung (17) ausgebildet ist.
13. Walzenanordnung (1) nach einem der vorangehenden Beispiele, wobei eine Walzenspaltdicke (D2) im im Wesentlichen parallel zu den Walzenachsen ausgebildeten Verfestigungsabschnitt (9) zwischen 0,01 mm und 0,03 mm, bevorzugt 0,02 mm beträgt.
14. Walzenanordnung (1) nach einem der vorangehenden Beispiele, wobei eine Walzenspaltdicke (D1) im ersten und/oder im zweiten konischen Verfestigungsabschnitt (8, 15) zwischen 0,02 mm und 0,08 mm, bevorzugt 0,05 mm beträgt.
15. Walzenanordnung (1) nach einem der vorangehenden Beispiele, wobei eine Höhe (H4) des ersten konischen Verfestigungsabschnitts (8) zwischen 0,2 mm und 0,6 mm, bevorzugt 0,4 mm beträgt.
16. Walzenanordnung (1) nach einem der Beispiele 10 bis 15, wobei eine Höhe (H5) des zweiten konischen Verfestigungsabschnitts (15) zwischen 0,2 mm und 0,6 mm, bevorzugt 0,4 mm beträgt.
17. Walzenanordnung (1) nach einem der vorangehenden Beispiele, wobei eine Breite (B2) des im Wesentlichen parallel zu den Walzenachsen ausgebildeten Verfestigungsabschnitts (9) zwischen 0,5 und 1 mm, bevorzugt 0,7 mm beträgt.
18. Walzenanordnung (1) nach einem der vorangehenden Beispiele, wobei ein Winkel (α) des ersten konischen Verfestigungsabschnitts (8) zur Vertikalen zwischen 10° und 18°, bevorzugt 14° beträgt.
19. Walzenanordnung (1) nach einem der Beispiele 10 bis 15, wobei ein Winkel (γ) des zweiten konischen Verfestigungsabschnitts (15) zur Vertikalen zwischen 10° und 18°, bevorzugt 14° beträgt.
20. Walzenanordnung (1) nach einem der Beispiele 11 bis 15, wobei der erste konische Verfestigungsabschnitt (8) parallel zum zweiten konischen Verfestigungsabschnitt (15) ausgerichtet ist.
21. Walzenanordnung (1) nach einem der vorangehenden Beispiele, wobei in der ersten und/oder in der zweiten Prägewalze (2, 3) eine Einrichtung zum Beheizen der Walzenoberfläche vorgesehen ist.
22. Wickelfähige, insbesondere vorverfestigte, Bahnware (22), aufweisend:
   zumindest eine sich in einer Ebene erstreckende wickelfähige Bahnwarenlage (23);
   eine Mehrzahl auf der zumindest einen Bahnwarenlage (23) verteilter Strukturelemente (24);
   wobei die Strukturelemente (24) jeweils eine das Strukturelement (24) zumindest abschnittsweise umgebende Umrandung (25) aufweisen, welche durch eine lokale Verfestigung der Bahnware (22) ausgebildet ist, wobei die Umrandung (25) zumindest einen ersten konischen Abschnitt (26) aufweist, dadurch gekennzeichnet, dass die Umrandung (25) ferner zumindest einen im Wesentlichen parallel zu der Ebene ausgebildeten Abschnitt (27) aufweist.
23. Bahnware (22) nach Beispiel 22, wobei die Umrandung (25) ein Stufenprofil aufweist.
24. Bahnware (22) nach Beispiel 22 oder 23, wobei der erste konische Abschnitt (26) unmittelbar an das Strukturmerkmal (24) angrenzt.
25. Bahnware (22) nach einem der Beispiele 22 bis 24, wobei der im Wesentlichen parallel zu der Ebene ausgebildete Abschnitt (27) unmittelbar an den ersten konischen Abschnitt (26) angrenzt.
26. Bahnware (22) nach Beispiel 25, wobei der erste konische Abschnitt (26) das Strukturelement (24) ringförmig umschließt.
27. Bahnware (22) nach Beispiel 25, wobei der im Wesentlichen parallel zu der Ebene ausgebildete Abschnitt (27) den ersten konischen Abschnitt (26) abschnittsweise umgibt.
28. Bahnware (22) nach einem der Beispiele 22 bis 27, wobei die Strukturelemente (24) als Öffnungen ausgebildet sind.
29. Bahnware (22) nach einem der Beispiele 22 bis 27, wobei die Strukturelemente (24) als Erhebungen ausgebildet sind.
30. Bahnware (22) nach einem der Beispiele 22 bis 28, wobei die Umrandung (25) einen zweiten konischen Abschnitt (28) aufweist, welcher gegenüberliegend zum ersten konischen Abschnitt (26) an den im Wesentlichen parallel zu der Ebene ausgebildeten Abschnitt (27) angrenzt.
31. Bahnware (22) nach einem derBeispiele 22 bis 28, wobei die Bahnware ferner eine Mehrzahl sich von der Ebene wegerstreckende Erhebungen (29) aufweist.
32. Bahnware (22) nach einem der Beispiele 30 oder 31, wobei der zweite konische Abschnitt (28) ferner unmittelbar an eine Erhebung (29) angrenzt.

### Bezugszeichenliste

- 1: Walzenanordnung
- 2: erste Prägewalze
- 3: zweite Prägewalze
- 4: Walzenspalt
- 5: Vorsprung, Nadel, Prägeerhebungen
- 6: Vertiefung
- 7: Walzenspaltabschnitt
- 8: erster konischer Verfestigungsabschnitt
- 9: im Wesentlichen parallel zu den Walzenachsen ausgebildeter Verfestigungsabschnitt
- 10: Stufenprofil
- 11: konisch proximaler Flankenabschnitt der Nadeln
- 12: konischer Miindungsabschnitt der Vertiefung
- 13: im Wesentlichen parallel zu den Walzenachsen ausgebildeter Sattelbereich des Sockelbereichs
- 14: im Wesentlichen parallel zu den Walzenachsen ausgebildeter Schulterbereich der Vertiefung
- 15: zweiter konischer Verfestigungsabschnitt
- 16: Prägeerhebungen
- 16.1: Prägeerhebungen mit erstem Querschnitt
- 16.2: Prägeerhebungen mit zweitem Querschnitt
- 17: Ausnehmungen
- 17.1: Ausnehmungen mit erstem Querschnitt
- 17.2: Ausnehmungen mit zweitem Querschnitt
- 18: konisch proximaler Flankenabschnitt der Prägeerhebung
- 19: konischer Miindungsabschnitt der Ausnehmung
- 20: Nadelspitze
- 21: Sockelbereich
- 22: Bahnware
- 23: Bahnwarenlage
- 24: Öffnung
- 25: Umrandung
- 26: erster konischer Abschnitt
- 27: im Wesentlichen parallel zu der Ebene ausgebildeter Abschnitt
- 28: zweiter konischer Abschnitt
- 29: Erhebung
- B1: Breite Vertiefung
- B2: Breite des im Wesentlichen parallel zu den Walzenachsen ausgebildeten Abschnitts
- B3: größte Breite der Nadeln
- D1: konische Prägespaltdicke
- D2: horizontale Prägespaltdicke
- H1: Höhe Nadel
- H2: Höhe Nadelspitze
- H3: Höhe Prägeerhebung
- H4: Höhe konisch proximaler Flankenabschnitt der Prägeerhebung
- H5: Höhe konischer Mündungsabschnitt der Ausnehmung
- T1: Tiefe der Vertiefung
- T2: Tiefe der Ausnehmung
- α: Winkel des konisch proximalen Flankenabschnitts der Nadeln
- β: Winkel Nadelspitze
- γ: Winkel konischer Mündungsabschnitt der Vertiefung

## Patentansprüche

1. Walzenanordnung (1) zur Veredelung einer wickelfähigen, insbesondere vorverfestigten Bahnware, aufweisend
eine erste und eine zweite Prägewalze (2, 3), welche einen Walzenspalt (4) zum Prägen der Bahnware zwischen sich ausbilden;
wobei die erste Walze (2) eine Mehrzahl Vorsprünge (5) zum Erzeugen von Strukturelementen in der Bahnware und die zweite Walze (3) eine Mehrzahl Vertiefungen (6) zum Aufnehmen der Vorsprünge (5) aufweist;
wobei ein zwischen der ersten und der zweiten Prägewalze (2, 3) gebildeter Walzenspaltabschnitt (7) zum Erzeugen einer das Strukturelement (24) jeweils zumindest abschnittsweise umgebenden Umrandung aus lokal verfestigter Bahnware ausgebildet ist, wobei der Walzenspaltabschnitt (7) zumindest einen ersten konischen Verfestigungsabschnitt (8) aufweist, **dadurch gekennzeichnet, dass** der Walzenspaltabschnitt (7) ferner zumindest einen im Wesentlichen parallel zu den Walzenachsen ausgebildeten Verfestigungsabschnitt (9) aufweist.

2. Walzenanordnung (1) nach Anspruch 1, wobei
(i) der Walzenspaltabschnitt (7) ein Stufenprofil (10) aufweist,
(ii) der erste konische Verfestigungsabschnitt (8) zwischen einem konischen proximalen Flankenabschnitt (11) der Vorsprünge (5) und einem konischen Mündungsabschnitt (12) der Vertiefung (6) ausgebildet ist, und/oder
(iii) der im Wesentlichen parallel zu den Walzenachsen ausgebildete Verfestigungsabschnitt (9) zwischen einem im Wesentlichen parallel zu den Walzenachsen ausgerichteten Sattelbereich (13) der Vorsprünge (5) und einem im Wesentlichen parallel zu den Walzenachsen ausgerichteten Schulterbereich (14) der Vertiefung (6) ausgebildet ist.

3. Walzenanordnung (1) nach einem der vorangehenden Ansprüche, wobei der erste konische Verfestigungsabschnitt (8) unmittelbar an den im Wesentlichen parallel zu den Walzenachsen ausgebildeten Verfestigungsabschnitt (9) angrenzt, wobei optional
(a) der erste konische Verfestigungsabschnitt (8) das Strukturelement (5) ringförmig umschließt, oder
(b) der im Wesentlichen parallel zu den Walzenachsen ausgerichtete Verfestigungsabschnitt (9) den ersten konischen Verfestigungsabschnitt (8) abschnittsweise umgibt.

4. Walzenanordnung (1) nach einem der vorangehenden Ansprüche, wobei
(i) die Vorsprünge (5) Nadeln sind, welche zum Erzeugen von als Öffnungen ausgebildeten Strukturelementen vorgesehen sind, oder
(ii) die Vorsprünge (5) Prägeerhebungen (16) sind, welche zum Erzeugen von als Erhebungen ausgebildeten Strukturelementen vorgesehen sind.

5. Walzenanordnung (1) nach einem der vorangehenden Ansprüche, wobei
(a) der Walzenspaltabschnitt (7) ferner einen zweiten konischen Verfestigungsabschnitt (15) aufweist, welcher gegenüberliegend zum ersten konischen Verfestigungsabschnitt (8) an den im Wesentlichen parallel zu den Walzenachsen ausgebildeten Verfestigungsabschnitt (9) angrenzt, wobei sich der erste und der zweite konische Verfestigungsabschnitt (8, 15) in entgegengesetzten Richtungen vom im Wesentlichen parallel zu den Walzenachsen ausgebildeten Verfestigungsabschnitt (9) wegerstrecken, und/oder
(b) die zweite Walze (3) eine Mehrzahl Prägeerhebungen (16) zum Erzeugen von Erhebungen in der Bahnware und die erste Walze (2) eine Mehrzahl Ausnehmungen (17) zum Aufnehmen der Prägeerhebungen (16) aufweist.

6. Walzenanordnung (1) nach Anspruch 5, wobei der zweite konische Verfestigungsabschnitt (15) zwischen einem konischen proximalen Flankenabschnitt (18) der Prägeerhebung (16) und einem konischen Mündungsabschnitt (19) der Ausnehmung (17) ausgebildet ist.

7. Walzenanordnung (1) nach einem der vorangehenden Ansprüche, wobei
(i) eine Walzenspaltdicke (D2) im im Wesentlichen parallel zu den Walzenachsen ausgebildeten Verfestigungsabschnitt (9) zwischen 0,01 mm und 0,03 mm, bevorzugt 0,02 mm beträgt,
(ii) eine Walzenspaltdicke (D1) im ersten und/oder im zweiten konischen Verfestigungsabschnitt (8, 15) zwischen 0,02 mm und 0,08 mm, bevorzugt 0,05 mm beträgt, und/oder
(iii) eine Höhe (H4) des ersten konischen Verfestigungsabschnitts (8) zwischen 0,2 mm und 0,6 mm, bevorzugt 0,4 mm beträgt.

8. Walzenanordnung (1) nach einem der Ansprüche 5 bis 7, wobei eine Höhe (H5) des zweiten konischen Verfestigungsabschnitts (15) zwischen 0,2 mm und 0,6 mm, bevorzugt 0,4 mm beträgt.

9. Walzenanordnung (1) nach einem der vorangehenden Ansprüche, wobei
(a) eine Breite (B2) des im Wesentlichen parallel zu den Walzenachsen ausgebildeten Verfestigungsabschnitts (9) zwischen 0,5 und 1 mm, bevorzugt 0,7 mm beträgt, und/oder
(b) ein Winkel (α) des ersten konischen Verfestigungsabschnitts (8) zur Vertikalen zwischen 10° und 18°, bevorzugt 14° beträgt.

10. Walzenanordnung (1) nach einem der Ansprüche 5 bis 9, wobei
(i) ein Winkel (γ) des zweiten konischen Verfestigungsabschnitts (15) zur Vertikalen zwischen 10° und 18°, bevorzugt 14° beträgt, und/oder
(ii) der erste konische Verfestigungsabschnitt (8) parallel zum zweiten konischen Verfestigungsabschnitt (15) ausgerichtet ist.

11. Walzenanordnung (1) nach einem der vorangehenden Ansprüche, wobei in der ersten und/oder in der zweiten Prägewalze (2, 3) eine Einrichtung zum Beheizen der Walzenoberfläche vorgesehen ist.

12. Wickelfähige, insbesondere vorverfestigte, Bahnware (22), aufweisend:
zumindest eine sich in einer Ebene erstreckende wickelfähige Bahnwarenlage (23);
eine Mehrzahl auf der zumindest einen Bahnwarenlage (23) verteilter Strukturelemente (24);
wobei die Strukturelemente (24) jeweils eine das Strukturelement (24) zumindest abschnittsweise umgebende Umrandung (25) aufweisen, welche durch eine lokale Verfestigung der Bahnware (22) ausgebildet ist, wobei die Umrandung (25) zumindest einen ersten konischen Abschnitt (26) aufweist, **dadurch gekennzeichnet, dass** die Umrandung (25) ferner zumindest einen im Wesentlichen parallel zu der Ebene ausgebildeten Abschnitt (27) aufweist.

13. Bahnware (22) nach Anspruch 12, wobei
(i) die Umrandung (25) ein Stufenprofil aufweist,
(ii) der erste konische Abschnitt (26) unmittelbar an das Strukturmerkmal (24) angrenzt,
(iii) der im Wesentlichen parallel zu der Ebene ausgebildete Abschnitt (27) unmittelbar an den ersten konischen Abschnitt (26) angrenzt, wobei optional
(a) der erste konische Abschnitt (26) das Strukturelement (24) ringförmig umschließt, oder
(b) der im Wesentlichen parallel zu der Ebene ausgebildete Abschnitt (27) den ersten konischen Abschnitt (26) abschnittsweise umgibt.

14. Bahnware (22) nach einem der Ansprüche 12 oder 13, wobei
(i) die Strukturelemente (24) als Öffnungen ausgebildet sind, oder die Strukturelemente (24) als Erhebungen ausgebildet sind,
(ii) die Umrandung (25) einen zweiten konischen Abschnitt (28) aufweist, welcher gegenüberliegend zum ersten konischen Abschnitt (26) an den im Wesentlichen parallel zu der Ebene ausgebildeten Abschnitt (27) angrenzt, und/oder
(iii) die Bahnware ferner eine Mehrzahl sich von der Ebene wegerstreckende Erhebungen (29) aufweist.

15. Bahnware (22) nach Alternative (ii) und/oder (iii) des Anspruchs 14, wobei der zweite konische Abschnitt (28) ferner unmittelbar an eine Erhebung (29) angrenzt.
